# EUROPÄISCHE PATENTANMELDUNG

(11) **EP 1 495 741 A2**
(43) Veröffentlichungstag der Anmeldung: **12.01.2005**
(21) Anmeldenummer: 04405437.7
(22) Anmeldetag: 09.07.2004
(51) Int. Cl.: A61F 13/511, A61F 13/84

(54) **Unterhose für leichte Inkontinenz und deren Anwendung**

(30) Priorität: 11.07.2003 CH 12162003
(71) Anmelder: VIP Domotec S.A.R.L., 3364 Leudelange (LU)
(72) Erfinder: Hahn, Franz-Josef, 56235 Ransbach-Baumbach (DE)
(74) Vertreter: Gaggini, Carlo, Dipl.Ing.

(57) **Zusammenfassung**

Die vorliegenden Erfindung betrifft eine Unterhose für leichte Inkontinenz, bestehend aus mindestens drei Schichten verwobener oder freier Textilfasern, nämlich aus einer inneren Schicht, die in direktem Kontakt mit der Haut steht, und die aus weichem und schwammartigem Material besteht, aus einer Zwischenschicht mit hohem Absorptionsvermögen, und aus einer widerstandsfähigen und elastischen Aussenschicht, und die dadurch gekennzeichnet ist, dass die innere Schicht eine Komponente aus Biokeramikmaterial aufweist, die "biologische" Infrarotstrahlung ausstrahlt, nämlich Infrarot mit einer Wellenlänge "λ" zwischen 6 und 16 µm.

Die erfindungsgemässe Unterhose findet vorteilhafte Anwendung beispielsweise zur Linderung einer ganzen Reihe von Problemen, die mit den folgenden Krankheiten einhergehen: Verheilung von Wunden und Rückbildung von Ekzemen, Menstruationsschmerzen der Frau, Gesundheitszustand der weiblichen Harnröhre, durch Hämorrhoiden verursachte Schmerzen sowie Probleme von Personen, die mit Ileus (Krummdarm) oder Kolostomie (künstlichem Darmausgang) leben müssen sowie Krankheitserscheinungen, die mit einer Prostatavergrösserung einhergehen.

## Beschreibung

Die vorliegende Erfindung betrifft eine Unterhose für leichte Inkontinenz und deren Anwendung, bestehend aus einer Kombination von mindestens drei textilen Schichten gemäss dem Oberbegriff des Anspruchs 1, und deren Anwendung gemäss den Ansprüchen 5 bis 10.

Das Problem der Inkontinenz gehört zweifelsohne zu den unangenehmsten Unannehmlichkeiten, von dem ein grosser Prozentsatz der männlichen und der weiblichen Bevölkerung betroffen ist, vor allem, aber nicht nur, im fortgeschrittenen Alter.

Daher ist nicht verwunderlich, dass die Hersteller von Unterhosen für leichte Inkontinenz heute eine Vielzahl von Produkten auf den Markt bringen, denen allen gemeinsam ist, dass sie drei oder mehr textile Schichten enthalten, nämlich eine innere Schicht, die weich und schwammig mit der Haut direkt in Kontakt kommt, eine Zwischenschicht von Super-Mikrofasern mit hohem Absorptionsvermögen, und eine äussere Schicht, die normalerweise aus einer Mischung von Baumwolle, Polyester und anderen mehr oder weniger elastischen Fasern besteht. Dieser Aufbau der bekannten und in Gebrauch stehenden Unterhosen für Inkontinenz kann selbstverständlich variieren, sowohl in der Anzahl der Schichten, wobei beispielsweise vier Schichten vorgesehen sein können, und die zum Beispiel eine dünne Schicht aus elastischem Polyurethan zwischen der äusseren Schicht und der Schicht mit hohem Absorptionsvermögen umfasst, als auch hinsichtlich der Art und Qualität der Fasern, welche die einzelnen Schichten bilden. Von den Chemiefasern, die angewendet werden, sind Fasern aus Polypropylen, Polyamid, Polyurethan, Polyester, usw., bekannt, die in der Zusammensetzung der verschiedenen Schichten Verwendung finden, als alleinige Faserkomponente oder in Mischungen auch mit Baumwolle, damit von den spezifischen Eigenschaften der einzelnen Faserarten bestmöglich profitiert werden kann.

Alle diese Lösungen genügen den Anforderungen an Unterhosen für leichte Inkontinenz zu einem gewissen Grad; die vorliegende Erfindung setzt sich hingegen eine weitere Verbesserung der Zuverlässigkeit der bekannten Produkte zum Ziel, was dank der Anwendung neuester physikalischer Erkenntnisse erreicht wird, welche das Auffinden unerwarteter und überraschender Materialeigenschaften ermöglicht haben.

Dieses Ziel konnte dank den im kennzeichnenden Teil des Anspruchs 1 genannten Eigenschaften erreicht werden.

Die in den Textilschichten eingebrachten Biokeramikmaterialien zur Rückstrahlung der vom menschlichen Körper abgegebenen Infrarotstrahlung ermöglichen, Vorteile zu erreichen, die im Folgenden näher erklärt werden. Vorab sind jedoch einige Vorbemerkungen allgemeiner Natur anzubringen, damit die Funktionsweise der festgestellten Wirkung erklärt werden kann.

### Vorhandensein von biokeramischem Material im Faseranteil

### Langwelliges Infrarot (FIR = Far Infra Red)

Alle Gegenstände, deren Temperatur über dem absoluten Nullpunkt (-273°C) liegt, strahlen thermische Energie in Form von Infrarot-Strahlung ab.

Im Fall des menschlichen Körpers beträgt die mittlere Oberflächentemperatur 34°C, was einer langwelligen Infrarot-Strahlung (FIR) entspricht. Diese Strahlung bildet das Emissionsspektrum des menschlichen Körpers im Wellenlängenbereich von 8 bis 15 Mikron.

Setzt man sich Bestrahlung mit FIR mit Wellenlängen aus, die jenen der vom menschlichen Körper natürlicherweise abgestrahlten ähnlich sind, ergibt sich ein spürbare Harmonisierungswirkung, insbesondere auf die organische Substanz, die den grössten Teil des Menschen ausmacht, nämlich das Wasser.

Wasser ist mit 60 bis 70% des Körpergewichts das Hauptelement der Lebewesen. Es befindet sich im Blut, in den Lymph-Gefässen, in den interzellulären Räumen sowie in den Zellen selbst.

Zwei grosse Flüssigkeitsbereiche lassen sich unterscheiden: ein intrazellulärer Beeich (50% des Körpergewichts) und ein extrazellulärer Bereich (20% des Körpergewichts, wovon 15% in den Zwischenräumen und 5% Plasma). Diese beiden Bereiche, die durch die Zellmembrane getrennt sind, welche eine selektive Durchlässigkeit aufweist, harmonisieren die Verlagerung von Wasser und Elektrolyten.

Die organischen Verlagerungen des Wassers sind daher von grundlegender Bedeutung für die Aufrechterhaltung der biologischen Prozesse, insbesondere für die Erneuerung der Haut und der subkutanen Gewebe. Wo dieses Gleichgewicht gestört ist, zeigt sich als erstes Anzeichen oft eine Wasseransammlung in der Haut, mit Problemen der Entwässerung und von Ödemen.

Unser Organismus ist in seinem Gleichgewichtszustand in der Lage, die zur Bildung von freiem Wasser erforderliche Energie aufzubringen, doch kann diese Energie abnehmen, auf jeden Fall mit zunehmendem Alter.

Die Anwendung "biologischer" Infrarotstrahlung (mittlerer und langer Infrarot-Wellenlängen) ermöglicht, den tieferliegenden Geweben durch die Haut eine sanfte Energie zuzuführen, die zur Anregung der natürlichen Prozesse beiträgt, insbesondere der wasserbezogenen Prozesse, dank einer Wirkung der Art der "Resonanz-Absorption"; dank diesen Erscheinungen ergibt sich eine bessere Entwässerung der Gewebe, eine bessere Ausscheidung der Toxine und eine bessere Sauerstoffversorgung.

Sonnen-Exposition erlaubt die Aufnahme von etwa 20% der Infrarotstrahlung. Doch die Menschen sind nicht immer an der Sonne, und überdies gibt die Sonne auch Strahlung ab, die der menschlichen Gesundheit abträglich ist.

Eine besondere Kombination keramischer Materialien wurde gefunden, die gewöhnlich "biokeramisches" Material genannt wird, und die sich bestens eignet, "biologische" Infrarotstrahlung auszustrahlen, d.h. mit einer Wellenlänge "λ" von 6 bis 12 µm.

Solche Keramikpulver werden durch Mischen herkömmlichen Keramikmaterials mit verschiedenen Mineralien hergestellt, die bei sehr hohen Temperaturen unter Anwendung hochentwickelter Technologien oxydiert wurden. Die hauptsächlich verwendeten Mineralien sind: Siliziumoxyd (SiO₂), Aluminiumoxyd (Al₂O₂), Magnesiumoxyd (MgO), Titanoxyd (TiO₂).

Als "biokeramische" Materialien werden auch natürlich vorkommende Materialien verwendet, wie Zeolithe, wobei die physikalisch-chemischen Eigenschaften und die organische Verträglichkeit des verwendeten Materials zu überprüfen sind.

Zu bemerken ist ferner, dass die Abstrahlung langwelliger Infrarotstrahlung durch das biokeramische Material nicht bloss auf die Rückstrahlung der vom menschlichen Körper abgegebenen Strahlung auf die Haut beschränkt ist, sondern dass diese Abstrahlung auch auf der Umwandlung elektromagnetischer Strahlung irgendwelcher Wellenlänge, die von aussen einfällt, in langwellige Infrarotstrahlung beruht; die biokeramischen Materialien verhalten sich dabei wie ein "schwarzer Körper", dessen Plank-Kurve ein Maximum zwischen 6 und 14 µm aufweist.

Über die quantitativen Aspekte hinaus gilt es festzuhalten, wie biokeramische Materialien eine Gesamtheit von elektromagnetischen Wellen abgeben, die gerade dem Spektrum des langwelligen Infrarots entspricht, und die sich daher wie ein Spiegel für das vom menschlichen Körper abgestrahlten Infrarots verhalten, der die Infrarotstrahlung praktisch aufnimmt und sie mit der gleichen Wellenlänge wieder auf den Körper zurückstrahlt.

Jedes Molekül zeichnet sich durch eine "Eigenfrequenz-Schwingung" aus; im Fall des Wassermoleküls beträgt die dieser Schwingung entsprechende Wellenlänge 8 bis 10 µm, also gerade der Frequenz der "langwelligen Infrarotstrahlung", welche die Biokeramischen Materialien abgeben.

Wird das Wassermolekül der Wirkung solcher Strahlung ausgesetzt, so gerät es in "Resonanz" zwischen zwei aufeinanderfolgenden Schwingungen.

### Auswirkungen des "Resonanzeffekts"

Als Kind haben wir alle schon auf dem federnden Netz des Bettes gespielt und dabei die Schwingungsbewegung von Sprung zu Sprung aufgeschaukelt, d.h. einen Resonanzeffekt erzeugt. Dieser Effekt baute sich jedoch sofort ab, sobald ein zweites Kind auf das federnde Bett sprang und nicht synchron bzw. in Gegenphase mit dem ersten Kind hüpfte.

Analog geraten die Wassermoleküle in Resonanz, doch ist diese Bewegung durch die benachbarten Moleküle begrenzt.

Der Resonanzeffekt bewirkt dabei jedoch, dass eine gewisse Anzahl Wassermoleküle in ihre Komponenten aufgespaltet werden, wodurch die Ionisation des Wassers in Wasserstoff-Ionen and Hydroxyd-lonen erfolgt.

Diese Erscheinung wird gewöhnlich als "Aktivierung des Wassers" benannt. Die "Aktivierung des Wassers" bestimmt verschiedene Phänomene, die zu einigen Ansprüchen der vorliegenden Patentanmeldung führen.

### Günstige Einflüsse des "Resonanzeffekts" auf den menschlichen Körper bezüglich metabolischer Prozesse

Die Verwendung biokeramischer Materialien, die Infrarotstrahlung mit Wellenlängen zwischen 6 und 14 Mikron abgeben, wenn die Einstrahlung in direktem Kontakt mit der Haut erfolgt, trägt dazu bei, die Homöostase der Gewebe zu steigern. Die wesentlichste Wirkung besteht in einer besonderen Aktivierung der Wassermoleküle mit günstigen Wirkungen auf die Vergleichmässigung des Wasseraustausches zwischen den verschiedenen Flüssigkeitsbereichen. Dieser Einfluss verbessert den Gewebeersatz, die Versorgung mit Sauerstoff und Nährstoffen, die Ausscheidung der Abfallstoffe, der Toxine und des Kohlendioxyds.

Dieser Einfluss verbessert auch die Blutzirkulation, insbesondere der Mikrozirkulation (in den Kapillaren).

Im Fall der Unterhose für leichte Inkontinenz gemäss der vorliegenden Erfindung betreffen diese günstigen Einflüsse schnellere Heilung von Wunden, Rötungen und Ekzemen, die im Kontakt mit der inneren Schicht der Unterhose stehen.

Darüber hinaus bewirkt eine bessere Blutzirkulation, und somit eine bessere Zufuhr von Nährstoffen und von Sauerstoff, verbesserte Widerstandsfähigkeit gegen neue Erkrankungen, bis hin zu einer Verbesserung der Blutzirkulation beim Mann in den "corpora cavernosa" (Schwellkörpern) und somit zu verbesserter Sexualfunktion, und bei der Frau zu einer Linderung der Menstruationsschmerzen.

Ein weiterer Vorteil besteht in verbessertem Abbau der Cellulitis (Fettleibigkeit) dank der verbesserten Ausscheidung von (fettartigen) Lipid-Substanzen.

### Auswirkungen des "Resonanzeffekts" hinsichtlich antibakterieller, pilzabwehrender und geruchsvermindernder Wirkungen

Wie erwähnt bewirkt die langwellige Infrarotstrahlung, welche die biokeramischen Materialien abgeben, die Ionisierung von Wasser, wobei sich einige Moleküle aufspalten und Wasserstoff- und Hydroxyd-Ionen bilden.

Selbstverständlich geschieht dieser Vorgang sowohl im Innern des Körpers als auch ausserhalb, wo immer etwas Feuchtigkeit vorhanden ist.

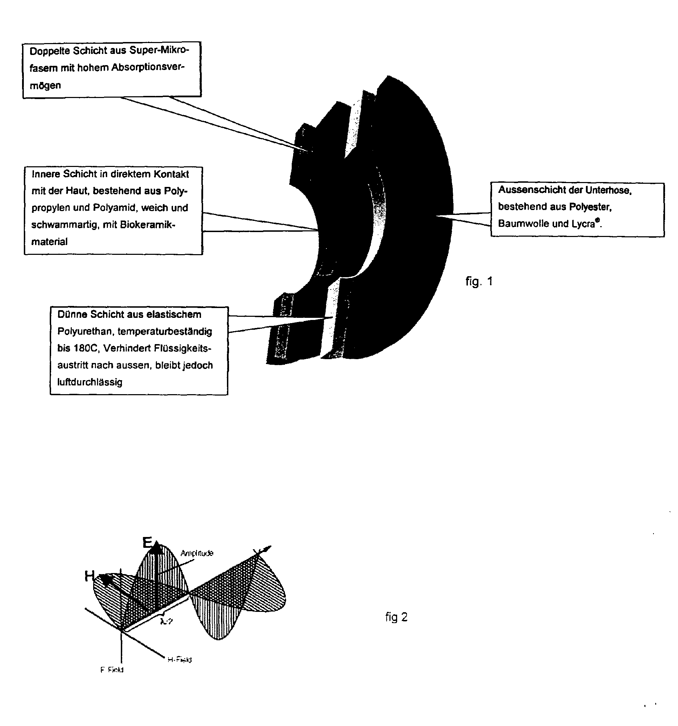

Zu erinnern ist auch daran, dass eine langwellige Infrarotstrahlung eine elektromagnetische Welle ist (Siehe die Fig. 2), und dass daher die damit einhergehende Leistung durch einen sogenannten "Poyntig-Vektor" dargestellt werden kann, dessen Richtung angibt, in welcher Richtung sich die Leistung verschiebt, und dessen Modul = "EH" den Betrag der Leistung darstellt, die der Welle zugeordnet ist, wobei "λ" die Wellenlänge angibt, "E" die elektrische Feldstärke und "H" die magnetische Feldstärke.

Wie irgendwelche Form von Primärenergie wandelt sich die Energie der elektromagnetischen Welle in Wärme um, sobald sie mit unserem Körper in Berührung kommt.

Diese Wärme ist es, die diesen molekularen Vorgang verursacht, der jenes Phänomen bestimmt, das "Aktivierung des Wassers" genannt wird, und diese Wärme bestimmt das "Aufspalten" von Toxinen und Bakterien sowohl im Innern des Körpers als auch auf der Epidermis (Aussenhaut).

Die Ausschaltung der Bakterien, und auf jeden Fall die Ausbildung einer Umgebung, die dank der Wärme gegen die Fortpflanzung der Bakterien wirkt, ist eine Erscheinung, die an sich leicht erklärlich ist, wenn man sich erinnert, wie unser Körper reagiert, wenn wir eine "Grippe kriegen": Die Temperatur steigt von den normalen 36.6°C auf 37.5 bis 38°C. Der Grund für diese Temperaturerhöhung beruht darauf, dass der grösste Teil der Bakterien abstirbt, wenn sie über eine bestimmte Zeit einer Temperatur von 37.5 bis 38°C ausgesetzt sind.

Somit erklärt sich, weshalb die langwellige Infrarotstrahlung eine antiseptische Wirkung hat, und es wird daher klar, weshalb sie keine Entstehung schlechter Gerüche erlaubt.

### Funktionsprinzip des Textilmaterials gemäss der vorliegenden Patentanmeldung und Überwindung der Begrenzungen gemäss dem Stand der Technik

Dank der Unterhose gemäss der vorliegenden Patentanmeldung wird es möglich, von den in den vorhergehenden Abschnitten erläuterten Vorteilen zu profitieren. Die innere Schicht der Unterhose, die in direktem Kontakt mit der Haut steht, wird unter Verwendung von Polymeren (beispielsweise Polyester) hergestellt, in welche während der Filamentherstellung biokeramische Materialien eingebracht werden, welche die vorher beschriebene Auswirkung auf die FIR ausüben, nämlich die Rückstrahlung der vom menschlichen Körper ausgehenden Infrarotstrahlung, dank dem Vorhandensein der biokeramischen Materialien.

Gemäss einer ersten Verbesserung der Erfindungsidee ist sodann vorgesehen, dass die normale hygienisierende Wirkung, die von der langwelligen Infrarotstrahlung ausgeht, gesteigert wird, indem im keramischen Material Ionen von Silber oder Silber-Zirkon, oder von anderen Metallen eingebracht werden, welche die Eigenschaft aufweisen, in Gegenwart von Feuchtigkeit Ionen abzugeben, welche die Bildung von aktivem Sauerstoff bewirken.

Die Versuche haben gezeigt, dass die Menge der abgegebenen Ionen in der Grössenordnung von einigen Teilen pro Milliarde (ppb) liegt. Solche Ionen wirken stark antibakterielle: Diese Wirkung beruht auf der von den metallischen Ionen hervorgerufenen Erzeugung von aktivem Sauerstoff. Die Versuche bestätigen, dass eine Umgebung wie die beschriebene besonders wirksam ist gegen viele Bakterien, einschliesslich Salmonellen.

### Resultate der Versuche betreffend antibakterielle Wirkung (*)

| ***Versuche mit Klebsiella Pneumoniae*** | | | |
|---|---|---|---|
| | Anfangsverschmutzung zur Zeit "0" (Anzahl Mikroorganismen) | Verschmutzung nach "18 Stunden" (Anzahl Mikroorganismen) | Verschmutzung in Prozenten |
| Baumwolle | 130'000 | 5'900'000 | + 4.438% |
| Standard Mikrofaser | 130'000 | 4'500'000 | + 3.361% |
| Mikrofaser mit Biokeramik-Materialmit eingebrachten Metall-Ionen (Silber, Silber-Zirkon od ähnliche Metalle) | 130'000 | < 10 | -99.99% |

| ***Versuche mit Staphylococcus Aureus*** | | | |
|---|---|---|---|
| | Anfangsverschmutzung zur Zeit "0" (Anzahl Mikroorganismen) | Verschmutzung nach "18 Stunden" (Anzahl Mikroorganismen) | Verschmutzung in Prozenten |
| Baumwolle | 140'000 | 6'700'000 | + 4.685% |
| Standard Mikrofaser | 140'000 | 5'700'000 | + 3.971% |
| Mikrofaser mit Biokeramik-Materialmit eingebrachten Metall-Ionen (Silber, Silber-Zirkon od ähnliche Metalle) | 140'000 | < 10 | -99.99% |
| (*) Durchführung der Versuche durch die unabhängigen Laboratorien IRM-Institut de Recherche Microbiologique - Rue Newton - S.I. Mitry-Compans-F-77290 Mitry-Mary - France | | | |

Die vorliegende Erfindung sieht sodann insbesondere eine ganze Reihe von Anwendungen bzw. Gebrauchsangaben vor, die im Folgenden beschrieben werden, und die Gegenstand abhängiger Patentansprüche sind.

Eine erste Anwendung ist jene zur Linderung der Menstruationsschmerzen der Frauen. Die Ursache der Menstruationsschmerzen beruht auf einer schlechten Blutzirkulation in der Gebärmutter (Uterus). Ist die Blutzirkulation nicht gleichmässig, so entsteht eine Muskelkontraktion bzw. Verspannung welche Schwellungen und Schmerzen verursacht.

Die langwellige Infrarotstrahlung steigert die Durchblutung des Uterus, was die Kontraktion zum Ausstossen des Abgangsmaterials unterstützt und überdies die Aktivität des Uterus aufrechterhält, so dass beim nächsten Mal die Menstruationsschmerzen weniger Unannehmlichkeiten verursachen.

Eine weitere Anwendung ist sodann jene, die sich dank verbesserter Mikrozirkulation des Blutes auf die Verbesserung des Gesundheitszustandes der weiblichen Harnröhre ausrichtet.

Im folgenden ist beschrieben, in welcher Weise dieser Mechanismus entsteht. Nicht selten passiert Frauen höheren und mittleren Alters, wenn sie niesen oder husten, oder wenn sie zu grosse Lasten tragen, dass ihnen einige Tropfen Urin entweichen; dies infolge Nachlassens der Spannung der Muskeln, welche die Harnröhre umschliessen, das dazu führt, dass meist etwas Harn abfliesst, wenn ein Druck auf die Harnröhre ausgeübt wird.

Ständiger Gebrauch der Unterhosen gemäss der vorliegenden Patentanmeldung verbessert die Widerstandsfähigkeit der Harnröhre dank der verbesserten Blutzirkulation, und entsprechend den vorher erwähnten Ausführungen beugt dies Infektionen vor, die zu Uretritis (Harnröhrenentzündung) oder Cystitis (Zystenbildung) führen können. Es ist übrigens nachgewiesen, dass solche Erkrankungen vom Verlust der Widerstandsfähigkeit gegen Mikro-Organismen der Vagina (Scheide) ausgehen.

Der ständige Gebrauch solcher Unterhosen ist entsprechend den vorhergehenden Ausführungen auch sehr nützlich zur Linderung der von Hämorrhoiden hervorgerufenen Schmerzen.

Die Entstehungsursache von Hämorrhoiden liegt in den langen Zeitspannen, während welchen wir sitzen, oft in Verbindung mit schlechten Ernährungsgewohnheiten. Dies verursacht eine Verlangsamung der Blutzirkulation (insbesondere der Mikrozirkulation) im Unterleib. In schweren Fällen kommt es zur Ruptur von Blutgefässen in der Aftergegend (Perianale Zone), die zu Koagulationen und schliesslich Bildung von Hämorrhoiden führt.

Die Wirkung der langwelligen Infrarotstrahlung beruht darauf, dass sie Koagulationen zum Verschwinden bringt, wodurch sich die Hämorrhoiden nach und nach zurückbilden.

Eine andere gemäss der vorliegenden Erfindung bevorzugten Anwendung bzw. Gebrauchsangabe ist sodann jene für die Verwendung durch Personen die an einem Krummdarm (lleum) leiden oder mit einer Kolostomie bzw. einem künstlichen Darmausgang leben.

Krankheitsbefunde wie "Intestinalkarzinom" (Darmkrebs) "Rectum Colon Ulcerosa" (Dünndarmgeschwür) und "Morbus Crohn" (Darmwandentzündung im untersten Dünndarm) erfordern nicht selten eine Resektion des betroffenen Darmabschnittes, wobei ein bleibender künstlicher Darmausgang auf der rechten oder linken Seite angelegt werden muss, je nach dem wie die Operation ausgeführt wurde.

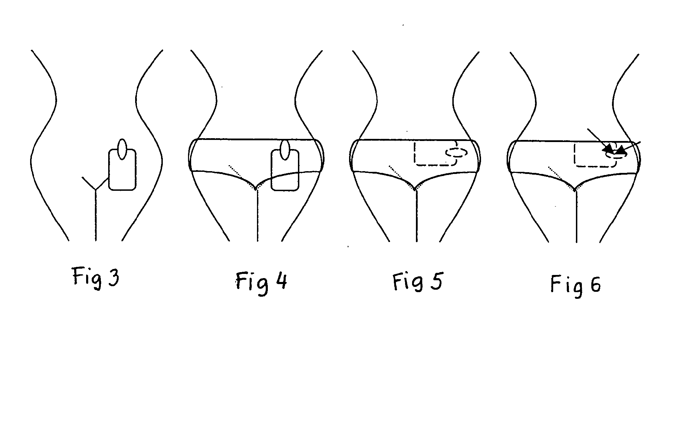

Weil dabei die Darmampulle am Darm-Ende, welche als Stauraum für die Abfallstoffe des Organismus dient, nicht mehr vorhanden ist, muss ein Sack zur Aufnahme der Fäkalien zu Hilfe genommen werden, wie dies in der Fig. 3 angedeutet ist.

Wenn die Unterhose angezogen wird, befindet sich der Sack in der in der Fig. 4 gezeigten Lage, wobei der Sack am künstlichen Darmausgang aus der Unterhose hinausreicht. Daher ist es ratsam, den Sack in horizontaler Lage zu positionieren, wie dies in der Fig. 5 dargestellt ist.

Ein schwerwiegender Faktor im Alltagsleben besteht darin, dass nicht selten Fäkalmaterial bei der Stelle, wo der Sack an die Kolostomie angefügt ist, wie dies in der Fig. 6 angedeutet ist. Insbesondere im Fall einer Resektion des Kolons, also des Organs, das unter den verschiedenen von diesem Darmteil ausgeübten Funktionen auch die "Drainage" der Flüssigkeiten übernimmt, ist das im Sack aufgefangene Fäkalmaterial im wesentlichen flüssig, und deshalb ist der Austritt flüssigen Materials an den in der Figur angezeigten Stellen noch häufiger.

Für Personen, die mit einem künstlichen Darmausgang leben müssen, ist es besonders wichtig, nicht sofort eingreifen zu müssen, sobald ein Vorfall wie der oben beschriebene eintritt, weil die Absorptionskapazität der Unterhose ausreichend Zeit sicherstellt, um sich zur Toilette bzw. ins Badezimmer zu begeben, um dort optimale Ordnung zu erstellen, womit die oft lähmende Angst vor der beschämenden Verlegenheit entfällt, plötzlich in mit Fäkalien beschmutzten Kleidern dazustehen.

Ein weiteres Schadenelement besteht darin, dass sich wegen des Fäkalienmaterials oft Risse in der peristomalen Zone zu bilden beginnen.

Somit ist klar, dass die Verwendung der Unterhose gemäss der vorliegenden Erfindung wie vorher beschrieben zwei Probleme löst:
- Sie hilft der Haut, sich zu vernarben bzw. zu regenerieren und Rötungen zum Verschwinden zu bringen (wobei festzuhalten ist, dass die langwellige Infrarotstrahlung nicht durch das Vorhandensein des Sackes am Stoma blockiert wird, sondern gleichwohl die Haut erreicht und ihre günstige Wirkung entfaltet);
- Dank der antibakteriellen Wirkung wird die Hygiene im Bereich verbessert und eine Vermehrung der Bakterien verhindert, mit entsprechend günstigen Auswirkungen auch unter dem Gesichtspunkt der entsprechenden Schmerzlinderung.

Die Unterhose gemäss der vorliegenden Erfindung ist sodann auch ratsam im Fall der Prostata-Vergrösserung des Mannes, wo sie viele dauerhafte Erleichterungen bringen kann.

Die unangenehme Vergrösserung der Prostata ist eine Erscheinung, die recht häufig ab dem 40 Lebensjahr auftritt, oft in Verbindung mit Ermüdungserscheinungen, Schwierigkeiten beim Harnlösen, und in schweren Fällen mit der Unmöglichkeit zu Urinieren.

Ebenso kann die Prostata-Vergrösserung zu einer Abnahme der Sexualfunktionen führen.

Von Prostata-Vergrösserung Betroffene leiden normalerweise an Prostatitis (Prostata-Entzündung), und in schwereren Fällen kann sich ein Prostatatumor entwickeln. Die vergrösserte Prostata drückt auf die Harnröhre und verursacht dadurch Schwierigkeiten beim Wasserlösen.

Medizinische und chirurgische Therapien sind oft eine Lösung, allerdings wirkt auch die langwellige Infrarotstrahlung auf die vergrösserte Prostata und deren Blutkoagulationen, die sich in ihrem Innern bilden, wobei die Blutzirkulation wieder vergleichmässigt und die Schmerzen reduziert werden.

Ebenfalls dank einer verbesserten Blutzirkulation in den "Corpora Cavernosa" (Schwellkörpern) ergibt sich eine spürbare Verbesserung der Sexualfunktionen. Ständige Behandlung der Prostata mit langwelliger Infrarotstrahlung durch Anziehen der erfindungsgemässen Unterhosen trägt somit zur Prävention gegen degenerative Krankheiten bei.

Im Folgenden ist im Sinn eines Beispiels der Aufbau einer Unterhose gemäss der vorliegenden Erfindung beschrieben, wobei zu unterstreichen ist, dass der grundsätzliche Aspekt bei der Realisierung der vorliegenden Erfindung nur darin liegt, dass Biokeramik-Material in einer oder mehreren der Textilschichten der Unterhose vorhanden ist. Die Zusammensetzung der Schichten, also die verwendeten Faserarten, die Anzahl der Schichten, usw., sind variable Parameter, die bei der Realisierung des Gegenstandes der vorliegenden Erfindung alle in Betracht kommen können.

Eine typische erfindungsgemässe Unterhose ist beispielsweise in folgender Art aufgebaut:
- Faser-Zusammensetzung: 64% Baumwolle + 24% Polyester + 6% Elastomer + 3% Polypropylen + 3% Nylon mit Biokeramikmaterial, wobei die beiden letztgenannten Komponenten der inneren Textilschicht in direktem Kontakt auf der Haut aufliegen.
- Bei "nahtloser" Herstellung mittels Santen-Maschinen schmiegt sich die Unterhose wie eine zweite Haut an und passt sich der Form des Körpers an, ohne sich bemerkbar zu machen, selbst wenn sie unter schweren Kleidungsstücken getragen wird.
- Die Unterhose kann bei 90°C gewaschen werden, auch in der Waschmaschine, wobei ein zu intensives Ausschwingen zu vermeiden ist.
- Einfache und sofortige Aufnahme von bis zu 150 ml Flüssigkeit.
- Die direkt auf der Haut aufliegende Schicht entspricht dem neuesten "Stand der Technik" bezüglich Technologie und antiseptische Wirkung. Sie besteht aus einer weichen und absorbierenden Textilschicht, die aus 50% Nylon mit Biokeramikmaterial und 50% Polypropylen besteht (Weiche und antiseptische Textilschicht, die dank ihrer Retentionsfähigkeit die Feuchtigkeit an die beiden inneren Schichten weiterleiten, die sich durch hohes Absorptionsvermögen auszeichnen).
- Das Absorptionsvermögen bleibt selbst nach Hunderten von Waschprozessen unverändert: dabei sollen keine Weichmacher irgendwelcher Art verwendet werden, weil dadurch die typischen Eigenschaften des Materials beeinträchtigt werden könnten, das ohnehin schon weich ist und keinerlei Weichmacher erfordert.
- Die Unterhosen sind vorgewaschen; deshalb ist keinerlei Wäsche vor dem Gebrauch erforderlich.
- Anordnung der Schichten, von aussen beginnend nach innen:
   - Aussenschicht der Unterhose, bestehend aus Polyester, Baumwolle und Lycra ® .
   - eine dünne Schicht aus elastischem Polyurethan, temperaturbeständig bis 180°C. Verhindert das Austreten von Flüssigkeit nach aussen, behindert jedoch den Luftdurchtritt nicht.
   - Doppelte Schicht aus Super-Mikrofasern von hohem Absorptionsvermögen.
   - Innere Schicht in direktem Kontakt mit der Haut, bestehend aus Polypropylen und Polyamid, weich und schwammartig, mit Biokeramikmaterial.

Die Abbildungen zeigen in der:
- Fig. 1 Die grundsätzliche Zusammensetzung einer Unterhose für leichte Inkontinenz gemäss der vorliegenden Erfindung,
- Fig. 2 Die elektromagnetische Welle eines langwelligen Infrarotstrahls,
- Fig. 3 bis 6 Die Verhältnisse bei einer Person, die sich einer Darmresektion unterziehen musste und daher mit einem künstlichen Darmausgang leben muss.

Die vorliegenden Erfindung ermöglicht es, eine ganze Reihe von Problemen zu beheben oder mindestens zu erleichtern, denen Personen mit Diskfunktionen des Urogenitaltrakts bzw. des ganzen Intestinaltraktes. unterworfen sind.

## Patentansprüche

1. Unterhose für leichte Inkontinenz, bestehend aus einer Kombination von mindestens drei Schichten von gewobenen oder freien Textilfasern, nämlich einer inneren Schicht, die in direktem Kontakt mit der Haut steht und aus einem weichen und schwammartigen Material besteht, einer Zwischenschicht mit hohem Absorptionsvermögen, und einer widerstandsfähigen und elastischen äusseren Schicht,
**dadurch gekennzeichnet, dass**
die innere Schicht eine Komponente aus Biokeramikmaterial aufweist.

2. Unterhose gemäss dem Anspruch 1,
**dadurch gekennzeichnet, dass**
in der Biokeramik-Komponente der inneren Schicht Ionen von Silber, Silber-Zirkon, oder von andern Ionen enthalten sind, die bewirken, dass in Gegenwart von Feuchtigkeit Ionen austreten, welche die Bildung von aktivem Sauerstoff hervorrufen und somit den normalen, dank der langwelligen Infrarotstrahlung ablaufenden lonisationsprozess verstärken.

3. Unterhose gemäss den Ansprüchen 1 oder 2,
**dadurch gekennzeichnet, dass**
die innere Schicht aus Polypropylen und Polyamid mit Biokeramikmaterial besteht, und dass die Zwischenschicht eine einfache oder doppelte Schicht aus Mikrofasern von hohem Absorptionsvermögen ist, und dass die Aussenschicht aus Polyester und/oder Baumwolle besteht.

4. Unterhose gemäss den Ansprüchen 1 bis 3,
**dadurch gekennzeichnet, dass**
zwischen der Aussenschicht und der Zwischenschicht auch eine dünne Schicht aus elastischem Polyurethan vorgesehen ist, welche das Austreten von Flüssigkeit nach aussen verhindert, aber den Luftdurchtritt erlaubt.

5. Anwendung der Unterhose gemäss den Ansprüchen 1 bis 4,
**dadurch gekennzeichnet, dass**
sie zur raschen Verheilung von Wunden und zur Reduktion von Ekzemen verwendet wird.

6. Anwendung der Unterhose gemäss den Ansprüchen 1 bis 4,
**dadurch gekennzeichnet, dass**
sie zur Linderung der Menstruationsschmerzen bei Frauen verwendet wird.

7. Anwendung der Unterhose gemäss den Ansprüchen 1 bis 4,
**dadurch gekennzeichnet, dass**
sie zur Verbesserung des Gesundheitszustandes der Harnröhre der Frau verwendet wird.

8. Anwendung der Unterhose gemäss den Ansprüchen 1 bis 4,
**dadurch gekennzeichnet, dass**
sie zur Linderung der durch Hämorrhoiden verursachten Schmerzen verwendet wird.

9. Anwendung der Unterhose gemäss den Ansprüchen 1 bis 4,
**dadurch gekennzeichnet, dass**
sie als Hilfsmittel für Personen verwendet wird, die mit einem lleus (Krummdarm) oder einer Kolostomie (künstlichem Darmausgang) leben.

10. Anwendung der Unterhose gemäss den Ansprüchen 1 bis 4,
**dadurch gekennzeichnet, dass**
sie zur Besserung der mit einer Prostatavergrösserung einhergehenden Krankheitserscheinungen verwendet wird.
